# EUROPEAN PATENT APPLICATION

(11) **EP 4 643 762 A1**
(43) Date of publication of application: **05.11.2025**
(21) Application number: 24186687.0
(22) Date of filing: 04.07.2024
(51) Int. Cl.: A61B 5/00, A61C 1/00, A61C 19/04

(54) **METHOD FOR DETERMINING ORAL HEALTH**

(30) Priority: 01.05.2024 US 202463641316 P
(71) Applicant: Koninklijke Philips N.V., 5656 AG Eindhoven (NL)
(72) Inventor: TARIDAL NAGANADA, Guruprakash, Eindhoven (NL)
(74) Representative: Philips Intellectual Property & Standards

(57) **Abstract**

The subject-matter of the present disclosure relates to a computer implemented method for determining oral health, and a system (300) thereof. The method involves receiving (401) audio data comprising audio of a tooth vibrating, analysing (402) the audio data to extract audio characteristics corresponding to an oral health condition, identifying (403) the oral health condition based on the extracted audio characteristics, and controlling (404) an electronic apparatus to output an indication of the oral health condition.

## Description

### FIELD OF THE INVENTION

The subject-matter of the present disclosure relates to computer implemented method for determining oral health, and a system in which the method may be performed. In particular, the present disclosure relates to a technique for determining tooth healthiness using a tool for cleaning teeth such as a toothbrush.

### BACKGROUND OF THE INVENTION

For oral health care to be effective, it is desirable to develop and maintain proper brushing guidance and habits - for example, the habit of brushing twice per day is deemed essential. However, for the brushing guidance to be effective, a proper correct diagnosis of the teeth health is paramount.

Existing electric toothbrushes offer various operational modes which can be selected based on the needs of the user: for example, if a user wants routine cleaning, the user can select a "clean" mode; if the user wants extra focus on cleaning, a "deep clean" mode may be selected; if the user has sensitive gums, a "sensitive" mode may be selected. Typically, changing the operating mode of an electric toothbrush involves changing parameters such as frequency of operation, length of operation, and so on.

However, optimal operation of the electric toothbrush generally requires an external determination of the oral health of the user, including, inter alia, identification of any issues for specific teeth. Such diagnosis is aided with evaluation of clinical radiographic examination, including anamnesis, inspection, palpation, and percussion. In addition to these examinations, some other clinical examinations like Plate index, Gingival index, Clinical attachment level and MB index are used in periodontal field.

More recently, techniques for indicating conditions such as dental caries have been developed which involve imaging light induced florescence to provide basic information about the dental caries. Many users consider camera based technologies to be too invasive (e.g., a threat to privacy), and such technology requires highly advanced image analysis capable of detecting and propose the personalised diagnosis and guidance.

It is therefore highly desirable to develop an alternative technique which can identify oral health in a quick and convenient way for the purposes of suggesting optimal oral hygiene procedures.

### SUMMARY OF THE INVENTION

The present invention is defined according to the claims.

According to a first aspect of the present invention, there is provided a computer implemented method for determining oral health. The method comprises receiving audio data comprising audio of a tooth vibrating (caused by touching the tooth with a vibrating part of an oral care device, such as a toothbrush), analysing the audio data to extract audio characteristics corresponding to an oral health condition, identifying the oral health condition based on the extracted audio characteristics, and controlling an electronic apparatus to output an indication of the oral health condition.

The described technique is a breakthrough for oral health. Sound analysis is a convenient way of collecting data from the brushing sessions of the users, while providing for robust output to provide users with confidence in the determination.

In an example, analysing the audio data may comprise transforming the audio data into frequency space. For example this may be a fast Fourier transform or a mel frequency cepstrum (and coefficients thereof).

In an example, identifying the oral health condition may be based on additional data comprising at least one of age of a user and a type of tooth corresponding to the received audio data.

In an example, the oral health condition may be at least one of healthy teeth, healthy gums, dental caries, cracked or broken teeth, and gum disease.

In an example, the indication of the oral health condition may be displayed to a screen of the electronic apparatus.

In an example, analysis of the audio data and identification of the oral health condition may be performed by a suitably trained machine learning model.

In an example, the method may further comprise generating a mouth map for a user in which indexes corresponding to a plurality of teeth of the user are stored in association with an identified oral health condition for a respective tooth of the user.

In an example, generating the mouth map comprise receiving a plurality of audio data corresponding to a plurality of teeth of the user, and performing the above method steps on each of the plurality of audio data.

In an example, generating the mouth map may comprise receiving locational data corresponding to a relative location of the plurality of teeth of the user (that is, a position of the tooth within the mouth) and displaying an image of the plurality of teeth positioned on the display based on the locational data. The locational data may comprise inertial measurement unit data received from the oral care device used to vibrate the tooth (and captured at the same time as capturing audio data).

In an example, the method may include displaying a recommended operational setting of the oral care device based on the identified oral health condition.

In a related aspect of the invention, there is provided a system for determining oral health. The system comprises an oral care device comprising a vibratable member, and an electronic apparatus comprising a microphone and a processor. The microphone is configured to receive audio data comprising audio of a tooth caused to vibrate using the vibratable member of the oral care device. The processor is configured to perform the method steps above.

In another related aspect of the invention, there is provided an alternative system for determining oral health. The system comprises an oral care device comprising a vibratable member, an electronic apparatus comprising a microphone, a first communicator, and a first processor, and a server comprising a second communicator and a second processor. The microphone is configured to receive audio data comprising audio of a tooth caused to vibrate using the vibratable member of the oral care device. The first processor is configured to control the first communicator to transmit the audio data to the server. The second processor is configured to perform the analysis and identification steps of the method above, and control the second communicator to transmit the identified oral health condition to the electronic apparatus. The first processor is configured to then control the electronic apparatus to output an indication of the oral health condition.

These and other aspects of the present invention will be apparent from and elucidated with reference to the embodiment(s) described hereinafter.

The techniques herein are not intended to replace proper, thorough, professional examination of oral health issues, but can be used as an indicator for oral health problems which may spur further professional examination.

### BRIEF DESCRIPTION OF DRAWINGS

The embodiments of the present inventions may be best understood with reference to the accompanying figures, in which:
Fig. 1 shows an example oral health device;
Fig. 2 shoes a system for determining an oral health condition;
Fig. 3 shows a method for determining an oral health condition from the system of Fig. 3;
Fig. 4 show example audio data captured for use in the method of Fig. 3;
Fig. 5 shows an example of transformed audio data used in the method of Fig. 3;
Fig. 6 shows another example of transformed audio data used in the method of Fig. 3;
Fig. 7 shows an example display for the output of the method of Fig. 3.

### DETAILED DESCRIPTION OF EMBODIMENTS

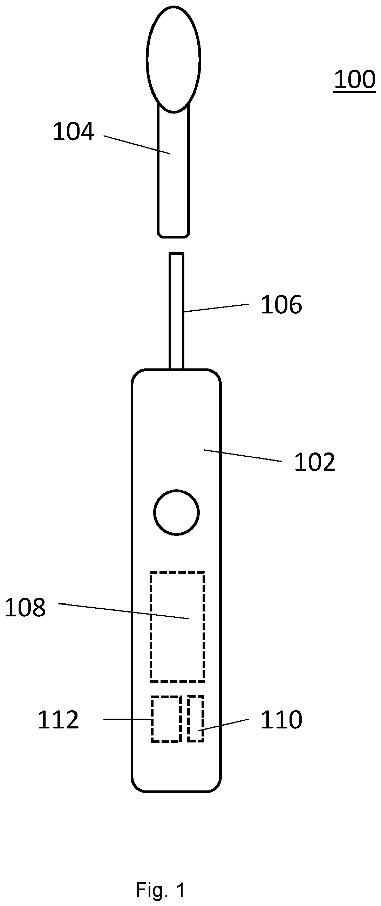

Fig. 1 shows an example of a personal care device. In the following example embodiments the personal care device is embodied as an oral care device 100, such as a toothbrush, which is generally used for the maintenance of oral health such as gum and teeth hygiene. It will however be appreciated that the example embodiments are not limited thereto and may be readily applied to any personal care device having the requisite features and functionality.

The oral care device 100 comprises a handle 102 and an operational head 104. The operational head may take a variety of forms depending on the intended usage of the oral care device 100. In these examples the operational head 104 takes the form of a brush head for brushing teeth, gums, and so on. The operational head 104 is a separate entity to the handle section 102 and is suitably configured to be driven by the handle 102 once attached. Suitably, the handle 102 comprises an engagement member 106 configured to insert into the operational head 104 to couple the head 104 to the handle 102.

The handle 102 also comprises a drive train assembly 108, housed inside the handle 102, which is configured to drive the operational head 104. As will be appreciated by those in the art, the drive train assembly 108 may comprise a motor or electromagnet(s) that generates oscillating movement of a spring assembly, which is subsequently transmitted to the operational head 104 via the engagement member 106 which extends from the drive train and acts as a drive shaft. Drive train assembly 108 can include components such as a power supply, an oscillator, and one or more electromagnets, among other components, as desired to perform its function of driving the operational head 104. Examples of drive train assemblies suitable for use in an oral care device can be found in commercially available sonic electric toothbrushes and are described in WO2015/101898, WO2018/046760, and WO2018/046429, all of which are incorporated by reference in their entirety.

Specifically, the drive train assembly 108 is configured to cause the engagement member to vibrate to drive particular motion of the operational head 104. As used herein, vibration encompasses reciprocating, oscillating, and/or rotational motions. Suitably, the engagement member 106 is also termed a vibratable member herein. It will be appreciated that the vibratable member 106 may take various different forms depending on the exact form of coupling required to the operational head 104. Preferably, however, the vibratable member 106 takes the form of an elongate structure coupled to the drive train assembly and protruding from an end of the handle section (e.g., a "top" end).

The handle 102 may also house a storage 110 configured to store drive profiles, or routines, for operation of the drive train 108. The drive profiles may set operating parameters for various modes of operation during a usage session, such as frequency and/or amplitude of vibration, duration of usage session, duration of operation at particular frequencies and/or amplitudes during a usage session, sequencing of particular frequencies and/or amplitudes during a usage session, and so on, and may be associated with a particular oral care requirement/procedure.

Suitably, a user may set an operating setting of the drive train assembly 108 based on one of these routines. In one example the setting may be selected via operation of a switch on the handle section 102. In another example, the setting may be selected using an external electronic apparatus, such as a mobile phone, and communicated to the oral care device 100; suitably the oral care device 100 may comprise a communicator 112, which may be housed within the handle 102, for communicating with an external apparatus. The communicator 112 may be configured to communicate wirelessly using one or more wireless communication protocols such as Bluetooth, ZigBee, WiFi, near field communication, and so on.

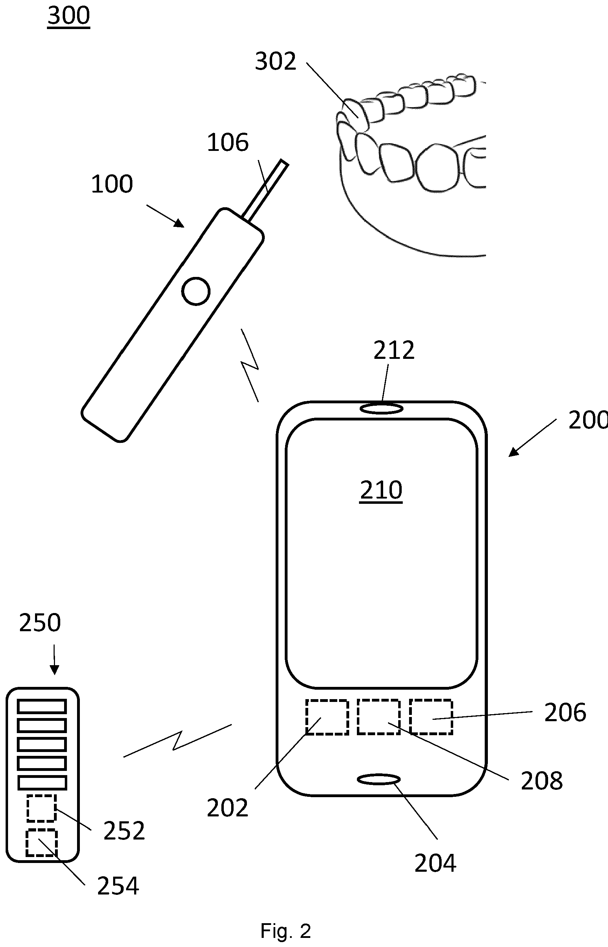

Fig. 2 shows a system 300 for determining oral health, which incorporates an oral health device 100 as outlined above. The system also comprises an electronic apparatus 200, which may be embodied as a smart device such as a mobile phone.

The apparatus 200 comprises a communicator 202 configured to communicate with the communicator 112 of the oral care device 100. The communication may allow for transfer of operational setting information to the oral care device 100, or indeed the receipt of current operational information from the oral care device 100 (such as a current operational profile setting).

The system comprises a microphone 204 which is configured to receive audio from the surrounding environment. In the illustrated example, the microphone 204 is provided on the apparatus 200, although in some examples the microphone 204 may be provided on a device external to the apparatus 200, such as on a microphone wand, or in some examples on the oral care device 100; in these alternative examples the external device/microphone may be configured to transmit captured audio to the apparatus 200.

When the system 300 is in use, the user of the oral care device 100 is directed to touch a part of the vibratable member 106 to one of their teeth 302, while the vibratable member 106 is operating. The vibration of the member 106 causes the tooth to vibrate generating a sound specific to the qualities of the touched tooth; more specifically, the vibrations/sound generated by the tooth are dependent on the health of the tooth and surrounding tissue in the mouth (e.g., gums). Suitably, it is audio corresponding to the sound of the tooth 302 vibrating which the microphone is configured to capture.

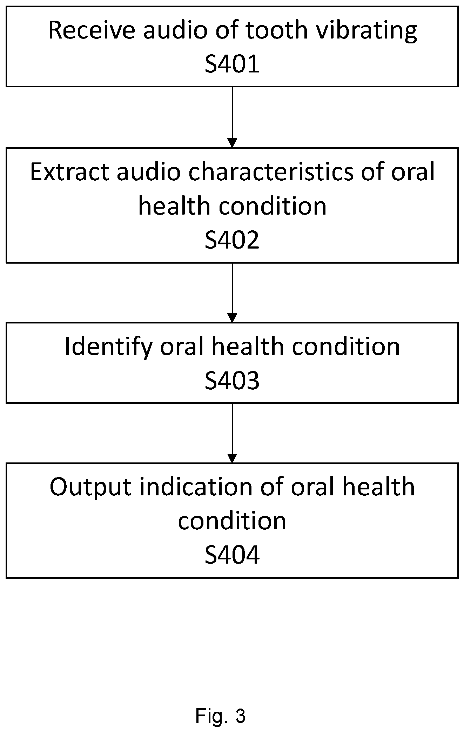

The apparatus 200 also comprises a processor 206. The processor 206 is configured to perform the computer implemented method shown in Fig. 3.

At step 401, the method comprises receiving audio data 304 of the tooth 302 vibrating. That is, the audio data captured by the microphone 204.

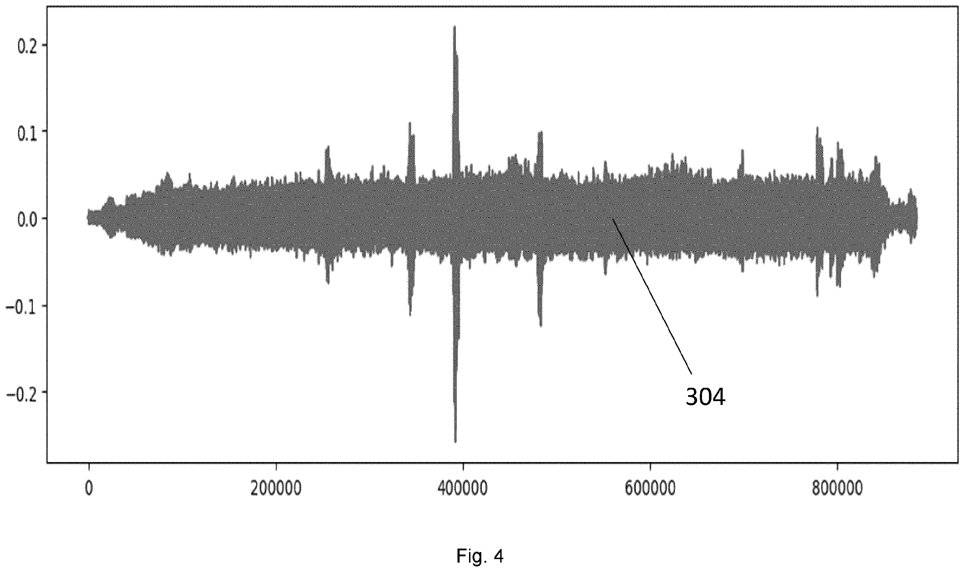

At step 402, the method comprises analysing the received audio data to extract audio characteristics corresponding to an oral health condition. The oral health condition may be one of healthy teeth, healthy gums, dental caries, cracked or broken teeth, and gum disease; although it will be appreciated that it may be possible to detect other oral health conditions using the techniques herein. An example of raw audio data captured by the microphone 204 is shown in Fig. 4. In particular, the example shown corresponds to a tooth with dental carries.

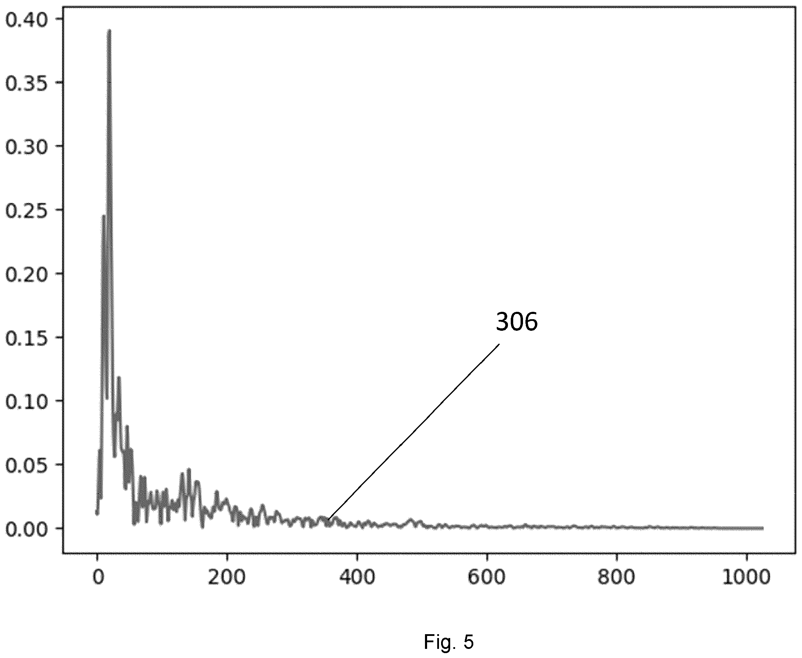

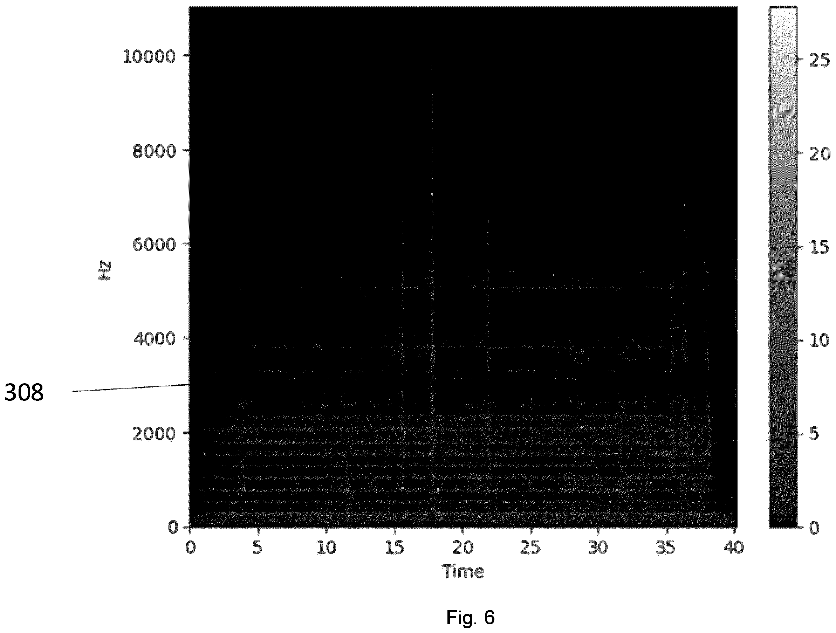

To analyse the audio data 304, it is preferred to transform the data into frequency space, in order to be able to better identify the relevant characteristics of the audio, as will be appreciated by those in the art. In one example, the audio data may be transformed using a fast Fourier transform 306. Fig. 4 shows an example of Fourier transforming the audio data of Fig. 4. That is, the example of Fig. 5 shows a Fourier transform of audio data (from a vibrating tooth 302) corresponding to dental caries. In another example, the audio data 304 may be transformed into a mel frequency cepstrum, as this allows for better extraction of the audio features for tooth health. Fig. 6 shows an example of deriving mel frequency spectrum coefficients 308 for the audio data of Fig. 4.

At step 403, the method comprises identifying the oral health condition based on the extracted audio characteristics. For each type of oral health condition, a certain form of audio output (and transform thereof) can be expected, so that the results of the audio analysis are characteristic of a particular class of oral health condition. That is, the inventors have determined that the audio generated from at least healthy teeth, healthy gums, dental caries, cracked or broken teeth, and gum disease, are sufficiently different to be detectable using suitable audio analysis techniques, particularly when suitably transformed as in either Figs. 5 & 6 to reveal relevant features in frequency space.

Moreover, identification of the oral health condition may also be based on a combination of the extracted audio characteristics with ancillary data. For example, different types of teeth may vibrate differently when touched by the vibrating member 106, therefore it may be desirable to account for tooth type (e.g., incisor, molar, etc); rather than specifying exact tooth type, a proxy parameter for tooth type may be used such as location within the mouth. In addition, some oral health conditions vary in severity and likelihood by age; therefore, it may be desirable to account for the age of the user when analysing and identifying an oral health condition. The ancillary data may be suitably stored on a storage 208 of the electronic apparatus 200, or optionally on an external server 250, and suitably retrieved by the processor 206 for use in the present method. The ancillary data may be provided by a user, for example via a guided user interface (GUI) shown on a display 210 of the apparatus 200.

It will be appreciated that feature extraction and identification of oral health condition may also be performed by a suitably trained machine learning model. In particular, it will be appreciated that the present techniques lend themselves to use of a classifier type machine learning model, where the input data is that of the sort shown in Figs. 5 & 6, and the output is one of healthy teeth, healthy gums, dental caries, cracked or broken teeth, and gum disease.

At step 404, the method comprises controlling the electronic apparatus 200 to output an indication of the oral health condition. In one example the output may be audible and suitably output via a speaker 212 of the apparatus 200. In another example, the indication is visual in nature, being suitably output to the display 210. That is, the processor 206 may control the display 210 to show what oral health condition has been determined to the user.

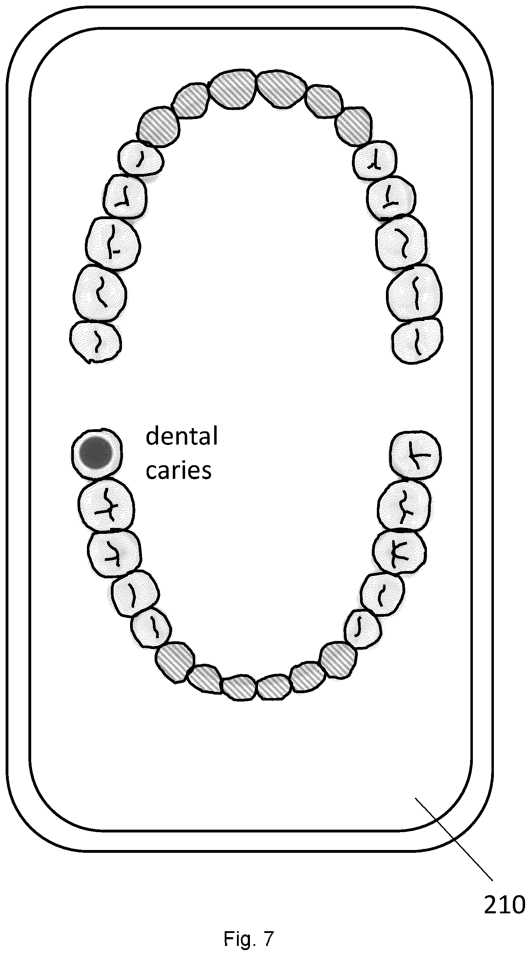

An example visual output is shown in Fig. 7. The visual output may show a visual indicator for each tooth of the user, and displays a different indicator for each type of determined oral health condition overlaid, or nearby, the corresponding tooth of the user. The system 300 may know which tooth the received audio data corresponds too by requesting such information from the user, either before or after initiating the steps of Fig. 3.

For example, a first type of visual indicator may be used to show teeth which are determined to have dental caries, a second type of visual indicator may be used to show teeth which are determined to be cracked or broken, a third type of visual indicator may be used to show teeth which are determined to have gum disease. It will be appreciated that the first to third indicators may correspond to teeth which have oral problems which may require an alteration to a dental care routine; on the contrary, where it is determined that a tooth has no oral health problems (so that the condition is healthy), then display of a tooth without any overlay markings may serve as the visual indicator for healthy teeth which do no require an alteration to the user's current dental care routine; alternatively, a fourth type of indicator may be used to show teeth which are healthy.

Optionally, at step 405, the method may comprise controlling to display a recommended operational setting of the oral care device 100 to the user. The method may also comprise transmitting a control command to the oral care device to begin operation of the drive train assembly 108 based on the recommended operational setting, according to an input of the user.

It will be appreciated that the method steps of Fig. 3 may be applied to a plurality of teeth of the user. In other words, Fig. 7 shows the generation of a mouth map for the user in which stores the oral health condition for a plurality of teeth of the user. More specifically, generating the mouth map may comprise assigning an index to each tooth of the user, and storing (in the storage 208) a database in which the teeth indexes are stored in association with the identified oral health condition for that respective tooth. Suitably, generating the mouth map for the user may comprise receiving a plurality of audio data corresponding to the users teeth, which may either be received in sequence (i.e., audio received, then analysed substantially immediately) or received in one go, separated, and then analysed.

Generating the mouth map may also comprise receiving locational data for each tooth, so that the display shown in Fig. 7 may be automatically generated without requiring input from the user. The locational data may take the form of inertial measurement unit (IMU) data captured by the oral care device 100 which is transmitted to the apparatus 200. For example, the IMU data may correspond to IMU data of a user tapping the vibrating member 106 to at least a front side and a back side of a tooth. The IMU data may be captured and transmitted to the apparatus 200 at substantially the same time as the microphone 204 is capturing the audio data. The oral care device 100 may be instructed to transmit the captured IMU data upon receipt of a controlling instruction from the apparatus 200.

As an alternative to the steps of Fig. 3 being performed by the apparatus 200, the method steps may instead be performed by a remote electronic device, such as the server 250. Here the processor 206 may be regarded as a first processor, and the communicator 202 as a first communicator. The server 250 suitably comprises a second communicator 252, so that the server 250 and apparatus 200 may communicate via the first and second receiver, and a second processor 254. The first processor 206 is configured to control the first communicator to transmit the received audio data to the server 250, with the second processor is configured to perform the steps 401 to 403. The second processor 254 then controls the second communicator 252 to transmit the identified oral health condition to the electronic apparatus 100, so that the first processor performs step 404 to output the indication of the oral health condition.

In summary then, a new technique for determining oral health using existing oral care devices has been provided.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measured cannot be used to advantage. Any reference signs in the claims should not be construed as limiting the scope.

At least some of the example embodiments described herein may be constructed, partially or wholly, using dedicated special-purpose hardware. Terms such as 'component', 'module' or 'unit' used herein may include, but are not limited to, a hardware device, such as circuitry in the form of discrete or integrated components, a Field Programmable Gate Array (FPGA) or Application Specific Integrated Circuit (ASIC), which performs certain tasks or provides the associated functionality. In some embodiments, the described elements may be configured to reside on a tangible, persistent, addressable storage medium and may be configured to execute on one or more processors. These functional elements may in some embodiments include, by way of example, components, such as software components, object-oriented software components, class components and task components, processes, functions, attributes, procedures, subroutines, segments of program code, drivers, firmware, microcode, circuitry, data, databases, data structures, tables, arrays, and variables. Although the example embodiments have been described with reference to the components, modules and units discussed herein, such functional elements may be combined into fewer elements or separated into additional elements.

## Claims

1. A computer implemented method for determining oral health, comprising:
receiving (401) audio data (304) comprising audio of a tooth (302) vibrating;
analysing (402) the audio data to extract audio characteristics corresponding to an oral health condition;
identifying (403) the oral health condition based on the extracted audio characteristics; and
controlling (404) an electronic apparatus to output an indication of the oral health condition.

2. The method of claim 1, wherein analysing the audio data to extract audio characteristics comprises transforming the audio data into frequency space.

3. The method of claim 2, wherein analysing the audio data to extract audio characteristics comprises analysing a fast Fourier transform (306).

4. The method of claim 2, wherein analysing the audio data to extract audio characteristics comprises analysing a mel frequency cepstrum (308).

5. The method of any preceding claim, wherein identifying the oral health condition is further based on additional data comprising at least one of age of a user and a type of tooth corresponding to the received audio data.

6. The method of any preceding claim, wherein identifying the oral health condition based on the extracted audio characteristics comprises identifying at least one of healthy teeth, healthy gums, dental caries, cracked or broken teeth, and gum disease.

7. The method of any preceding claim, wherein controlling the electronic apparatus to output an indication of the oral health condition comprises controlling a display (210) of the electronic apparatus.

8. The method of any preceding claim, wherein at least one of the steps of analysing the audio data to extract audio characteristics and identifying the oral health condition based on the extracted audio characteristics, are performed using a trained machine learning model.

9. The method of any preceding claim, further comprising generating a mouth map for a user in which indexes corresponding to a plurality of teeth of the user are stored in association with an identified oral health condition for a respective tooth of the user.

10. The method of claim 9, wherein generating the mouth map comprises receiving a plurality of audio data corresponding to a plurality of teeth of the user, and performing the steps of claim 1 on each of the plurality of audio data.

11. The method of claim 9 or 10, wherein generating the mouth map comprises receiving locational data corresponding to a relative location of the plurality of teeth of the user, storing the locational data in association with the respective index for the plurality of teeth, and displaying an image of the plurality of teeth positioned on the display based on the locational data.

12. The method of claim 11, wherein the locational data comprises inertial measurement unit (108) data received from an oral care device.

13. The method of any preceding claim, further comprising controlling the electronic apparatus to display a recommended operational setting of an oral care device, based on the identified oral health condition.

14. A system (300) for determining oral health, comprising:
an oral care device (100) comprising a vibratable member (106);
an electronic apparatus (200) comprising a microphone (204) and at least one processor (206), wherein the microphone is configured to receive (401) audio data (304) comprising audio of a tooth (302) caused to vibrate using the vibratable member of the oral care device, and the processor (206) is configured to:
analyse (402) the received audio data to extract audio characteristics corresponding to an oral health condition,
identify (403) the oral health condition based on the extracted audio characteristics, and
control (404) the electronic apparatus to output an indication of the oral health condition.

15. A system (300) for determining oral health, comprising:
an oral care device (100) comprising a vibratable member (106),
an electronic apparatus (200) comprising a microphone (204), a first communicator (202), and a first processor (206), and
a server (250) comprising a second communicator (252) and a second processor (254), wherein:
the microphone (204) is configured to receive (401) audio data (304) comprising audio of a tooth (302) caused to vibrate using the vibratable member of the oral care device,
the first processor is configured to control the first communicator to transmit the audio data to the server,
the second processor is configured to analyse (402) audio data received via the second communicator to extract audio characteristics corresponding to an oral health condition, identify (403) the oral health condition based on the extracted audio characteristics, and control the second communicator to transmit the identified oral health condition to the electronic apparatus, and
the first processor is configured to control (404) the electronic apparatus to output an indication of the oral health condition.
